(19) Europäisches Patentamt / European Patent Office / Office européen des brevets

(11) **EP 2 175 740 B1**

(12) **EUROPEAN PATENT SPECIFICATION**

(45) Date of publication and mention
of the grant of the patent:
**06.06.2018 Bulletin 2018/23**

(21) Application number: **08785228.1**

(22) Date of filing: **30.07.2008**

(51) Int Cl.:
*A61J 3/02* *(2006.01)*    *A61J 3/10* *(2006.01)*
*A61K 9/16* *(2006.01)*    *A23P 10/25* *(2016.01)*
*A23G 3/34* *(2006.01)*    *A61K 9/20* *(2006.01)*

(86) International application number:
**PCT/EP2008/006283**

(87) International publication number:
**WO 2009/015880 (05.02.2009 Gazette 2009/06)**

(54) **DIRECT COMPRESSIBLE DEXTROSE**

DIREKT KOMPRIMIERBARE DEXTROSE UND DARAUS HERGESTELLTE TABLETTEN

DEXTROSE POUVANT ÊTRE SOUMISE À UNE COMPRESSION DIRECTE ET COMPRIMÉS LA COMPRENANT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **31.07.2007 EP 07015044**

(43) Date of publication of application:
**21.04.2010 Bulletin 2010/16**

(73) Proprietor: **Cargill, Incorporated**
**Wayzata, MN 55391 (US)**

(72) Inventors:
• **BRYS, Katleen, Rosa, François, Albert**
**B-2280 Bornem (BE)**

• **MEEUS, Liesbeth, Maria, Fernande**
**B-3078 Everberg (BE)**

(74) Representative: **Wibbelmann, Jobst**
**Wuesthoff & Wuesthoff**
**Patentanwälte PartG mbB**
**Schweigerstrasse 2**
**81541 München (DE)**

(56) References cited:
**EP-A- 1 108 792       US-A- 3 619 292**
**US-A- 4 072 535       US-A1- 2003 005 923**
**US-A1- 2005 075 335   US-A1- 2005 202 084**
**US-B1- 6 451 122      US-B2- 6 881 432**

**Description**

This invention relates to a process for preparing a direct compressible dextrose composition by compression and subsequent granulation, and a process for preparing pharmaceutical formulations or food formulations in tablet form using the direct compressible dextrose composition. Compressed tablets represent the most popular pharmaceutical dosage form with about one-half of all prescriptions dispensed for compressed tablets. There are currently three basic methods of commercially preparing compressed tablets. These are direct compression, dry granulation followed by compression, and wet granulation followed by drying and compression.

The direct compression method involves directly compacting a blend of active ingredient(s) and one or several inert materials, referred to as excipients, by means of a tablet press. This method offers the advantage of only a few production steps but also has many technological limitations. For example, direct compression is only employable if a sufficiently homogeneous mix can be obtained by simple mixing, the flow property of the mix is satisfactory, and the ingredients of the mix allow for direct compression.

In cases where direct compression cannot be used due to the nature of the used active substances or other ingredients, which is often the case, a method called granulation is used. Granulation is a process in which primary powder particles are made to form larger entities called granules. The granulation allows to prevent segregation of the constituents of the powder mix, to improve the flow properties of the powder mix, and to improve the compaction characteristics of the powder mix.

Granulation methods can be divided in two basic types, namely wet methods, which use a liquid in the process, and dry methods in which no liquid is used. Wet granulation is most often used and involves many steps, including: agglomerating of dry primary powder particles of active ingredients and excipients in the presence of a granulating fluid upon agitation using low-shear or high-shear mixers or fluidized beds, wet sieving to remove larger lumps, drying the granulation, and milling or sieving the dried granulation to achieve a granulation having the desired granule size distribution. The obtained granulation may subsequently be tabletted, capsulated or filled in sachets.

In dry granulation methods, the primary powder particles are aggregated under high pressure. In pharmacy, two methods are commonly used for dry granulation. Either a large tablet (known as a 'slug') is produced in a heavy-duty tablet press (a process known as 'slugging') or the powder is compacted between two rollers to produce so-called 'flakes' (a process known as 'roller compaction'). In both cases the intermediate products are broken using a suitable milling technique to produce a granular material, which is usually sieved to separate the desired size fraction. The dry granulation method is particular useful for active ingredients that do not compress well after wet granulation, or those that are moisture sensitive or thermo labile.

For dry granulation two different apparatus are necessary: first, an apparatus for compacting the dry powders into compacts ('compactor'), and secondly an apparatus for crushing these compacts into granules ('granulator'). In commercially available roller compactors the process of roller compaction and granulation, i.e. the crushing of the compacts into granules, are usually combined in a single compactor/granulator, commonly known as 'roller compactor'.

The compressed tablets produced by the above methods are usually composed of food ingredients (in the case of food-related compressed tablets) or one or more active ingredients (in the case of pharmaceutical compressed tablets) and several inert materials, referred to as excipients. The numerous tabletting excipients which are currently used for preparing tablets are generally classified according to their function, such as (i) diluents (also called bulking agents and fillers), e.g. lactose or sorbitol, which provide a quantity of material that can accurately be formed into a compressed tablet, (ii) binders, e.g. methyl cellulose, which hold the ingredients together in the compressed tablet, (iii) lubricants, e.g. magnesium stearate or polyethylene glycol, which improve the release of the compressed tablet from the die and punches of the tablet press, and (iv) disintegrants, e.g. starch or cellulose, which help facilitate the break-up of the tablet when placed in a fluid environment, and (v) other substances, such as emulsifiers and surfactants.

As mentioned above, when direct compression methods are used, the choice of excipients is highly critical. In particular, the active ingredients and the excipients need to be 'direct compressible', i.e. needs to have good flow properties and the compaction characteristics required to produce strong tablets. Excipients that are suited for direct compression are well-known in the art and include, for example, various sugars, such as lactose and sucrose, and sugar alcohols, such as sorbitol and mannitol.

Dextrose (D-glucose) in powder form is one of the sugars which have been used as a diluent in tablets formed by direct compression methods. Three crystalline forms of dextrose are commonly described. These are (i) $\alpha$ dextrose monohydrate, which is traditionally produced by slow crystallisation, and by cooling supersaturated syrups with a high glucose content originating from the hydrolysis of starch, (ii) anhydrous $\alpha$ dextrose, which is generally produced by dissolving crystals of $\alpha$ dextrose monohydrate in water at 60°C to 65°C, for example, in autoclaves under a high vacuum and under carefully controlled conditions, and (iii) anhydrous $\beta$ dextrose, which is typically produced by a crystallization process at a high temperature.

The above-mentioned single crystal forms of dextrose, however, do not show sufficient compressibility. It is generally accepted that the preparation of a dextrose in powder form having sufficient compressibility properties and suitable to prepare tablets with desirable properties, requires the mixing of α dextrose monohydrate with additives, such as maltose, maltodextrins or other polysaccharides with a higher degree of polymerisation (DP), or the production of mixed compositions of anhydrous α and β forms of dextrose, for example, by atomisation of a glucose syrup with a high dry matter content.

US 2005/020284 A1 discloses a pharmaceutical composition in the form of a chewable tablet for the suppression of gastric reflux comprising an alginic acid or salt thereof, a water-soluble carbonate radical precursor, a calcium salt, a first bulk sweetener, and a binding agent. In the process to render the calcium salt suitable for tabletting the calcium carbonate is granulated with a bulk sweetener which can be dextrose. The amount of bulk sweetener, however, is lower than 50% by weight.

US-A-4,072,535 teaches a free-flowing binder-disintegrant powder material consisting essentially of a precompacted-starch powder. According to this document, a dry granulation process may be utilized in preparing a direct compressible starch composition.

US 4,327,077 discloses that dextrose monohydrate can be used as a direct compaction vehicle in compressed chewable antacid tablets. In these antacid tablets the dextrose monohydrate acts as a binder for combined particles of recrystallized fatty material, such as chocolate, and an antacid. According to US 4,327,077, the used dextrose in the monohydrate form may be one of several mentioned, commercially available direct compressible dextrose preparations. These dextrose preparations, however, are known to contain 5% to 6% of maltose, maltotriose and maltodextrins with a higher DP, and are generally obtained by atomisation of a glucose syrup with a dextrose equivalent (DE) in the range of 93% to 99%.

Further, US 2002/0122823 A1 describes a tablet capable of being chewed or disintegrated in the oral cavity, which comprises a pharmaceutically active ingredient, and a matrix comprising direct compressible dextrose monohydrate and sucralose, wherein the table is substantially fat free and the matrix is substantially free of non-saccharide, water soluble polymeric binders. The method of production of the used 'direct compressible dextrose monohydrate', however, is not disclosed. US 2002/0122823 A1 only describes that the direct compressible dextrose is a dextrose in the monohydrate form having an average particle size of 100 to 500 μm, wherein this average particle size is said to be required to impart the formulation with adequate flowability and compressibility.

US 2005/0202084 A1 discloses a pharmaceutical composition in the form of a chewable tablet for the suppression of gastric reflux comprising inter alia a calcium salt. The calcium salt may be blended via any of wet granulation, spray drying, or compression processes prio to admixture with alginic acid and a carbonate radical precursor.

US 3,619,292 discloses so-called Massecuite Aggregated Total Sugar granules. These granules are employed as a binder or binder-filler in the manufacture of compressed tablets, preferably by direct compression of a particulate tableting composition into which the Total Sugar granules have been blended.

Another dextrose preparation in powder form with high compressibility and high dextrose content and purity, which is suited for the production of tablets by direct compression, is known from US 6,451,122 B1. The dextrose described therein is an anhydrous dextrose characterized by a dextrose content of at least equal to 99%, an anhydrous α crystalline form content of at least equal to 95%, a water content of at most equal to 1%, a compressibility, determined by a test A, of at least equal to 80 N, and a mean diameter in the range of 150 μm to 200 μm. This powdered dextrose is prepared by a rather complicated process comprising subjecting a starting dextrose, i.e. a dextrose powder with a high dextrose content and of essentially the α form, which can be obtained by crystallisation or atomisation, to a dry granulation process using water or glucose syrup as a binder.

In view of the above, there is still a need for a process producing a dextrose composition exhibiting excellent flowability, friability and/or compressibility suited for use as an ingredient in pharmaceutical or food formulations that is obtainable in a simple, cost-efficient and reproducible manner.

This need is met by the provision of a dry granulation process according to claim 1. This process comprises the steps of (a) providing a starting dextrose composition in powder form, (b) compacting the starting dextrose composition by (i) roller compaction using compacting rolls to form flakes or (ii) slugging using a tablet press of single-punch or rotary type to form slugs, (c) crushing the flakes or the slugs obtained in step (b) to form granules, and (d) screening the granules to obtain a direct compressible dextrose composition in the form of granules with a given particle size distribution.

It was surprisingly found that compression of conventional, commercial available dextrose monohydrate by means of roller compaction or slugging followed by granulation leads to a dextrose granulation that is highly suitable for use in, for example, sachets and capsules, and for forming tablets. In particular, the direct compressible dextrose composition of the invention allows to produce tablets with higher tablet hardness compared to conventionally used dextrose. This in turn enables the incorporation of more active ingredient(s) while maintaining a certain tablet hardness. It also makes it possible to manufacture smaller and 'nicer' tablet sizes while still providing a particular and sufficient dosage of the active ingredient. Furthermore, the direct compressible dextrose composition allows to prepare tablets having a certain hardness using a lower compression force. In addition, upon compressing the direct compressible dextrose composition into a tablet, no capping is observed. Finally, the friability of tablets produced using the direct compressible dextrose composition of the invention is lower and, thus, more resistant against shipment and abrasion on handling.

The starting dextrose composition used in the context of the present invention comprises at least 50% by weight dextrose, preferably at least 60%, 70% or 80% by weight dextrose, more preferably at least 90% by weight dextrose, and even more preferably 95% to 100% by weight dextrose. Most preferably the starting dextrose composition comprises at least 98%, at least 99%, or at least 99.5% by weight dextrose. The dextrose contained within the starting dextrose composition is $\alpha$ dextrose monohydrate, or a mixture of $\alpha$ dextrose monohydrate and anhydrous $\alpha$ dextrose, and/or anhydrous $\beta$ dextrose provided that the moisture content of the direct compressible dextrose composition is at least 2%, preferably 4%, more preferably 7%, or even between 7 to 12%, or up to 10 to 12%. According to the European Pharmacopoeia, dextrose monohydrate is containing between 7 to 9.5% water and to the US Pharmacopoeia between 7,5 to 9,5% water.

Substances, which may be included in the starting dextrose composition may, for example, comprise conventionally known excipients, such as diluents (bulking agents or fillers), binders, lubricants, disintegrants, and other compounds. For example, a lubricant, such as magnesium stearate and other related stearates, stearic acid, polyethylene glycol, sodium stearyl fumarate, hydrogenated castor or vegetable oil, talc or mixtures thereof in a total amount of 0.01 to 10% by weight, based on the weight of the total composition, may be present. Examples of other excipients are well-known in the art and/or mentioned herein below.

According to the present invention, the additional ingredients, if present, are homogenously blended to provide a homogeneous starting dextrose composition, which is subsequently compacted. Any conventional mixer, such as a ribbon mixer, or a blender, such as a twin shell blender, can be used for blending an ingredient mixture in powder form until a uniform mix is formed.

In a preferred embodiment of the invention, the step (b) of compacting the starting dextrose composition is carried out by roller compaction using at least two compacting rolls to form so-called flakes (a type of compact). The two compacting rolls rotate in opposite directions to draw the starting dextrose composition into the gap between the compacting rolls. The pressure imparted enhances the bonding forces between the solid surfaces of the powder particles of the starting dextrose composition and leads to the formation of the flakes. The roll pressure, roll gap and roll speed can readily be selected by a person skilled in the art to provide the desired characteristics of the flakes, such as strength and friability, and the desired properties of the granules obtained after crushing and screening the flakes, such as particle size distribution and flowability. The roll pressure, the most significant parameter of roller compaction, is preferably in the range of 30 to 150 bar, in particular in the range of 40 to 120 bar, and particularly preferred in the range of 50 to 100 bar. A preferred roll gap is 0.5 to 5 mm, more preferably 0.5 to 3.0 mm and most preferably 1 to 2 mm. Suitable roll speeds to maintain a steady output of material and to obtain flakes and granules, respectively, with the required properties are between 2 and 15 rpm, preferably between 3 and 10 rpm, particularly between 4 and 8 rpm.

The step of compacting the starting dextrose composition by roller compaction can be carried out by means of any commercially available roller compactor, having for instance straight or profilated rollers, vertical, horizontal or diagonal arrangements of the rollers, a roller cooling system, and a feed system with single or multiple-auger operation, optionally designed with a vacuum system. A particularly suited compactor for use within the present invention is roller compactor type WP 120 V Pharma of Alexanderwerk. Other suitable compactors are well-known in the art and may be, e.g., be obtained from Fitzpatrick Co. (United States), Vector Co. (United States), RIVA S.A. (Argentina), Freund Industrial Co. (United States), Gerteis AG (Germany), and Hutt GmbH (Germany).

As an alternative, the compression may also be carried out by a process called 'slugging' since it is well know that, under appropriate conditions, the slugging end product is largely the same as the one obtained by roller compaction. Slugging involves the compression of the starting dry dextrose composition into larger tablets or 'slugs' by means of a conventional tablet machine or, more usually, a specially designed tablet press, for example a tablet press of single-punch or rotary type, in particular a large heavy-duty rotary press.

After compacting, the obtained flakes or slugs are crushed to form granules and subjected to screening to reduce in size. The granulation may occur in a single-stage or a multi-stage granulating process. In other words, the flakes or slugs may be first crushed into smaller particles called granules which are subsequently screened to obtain a direct compressible dextrose composition in the form of granules with a given particle size distribution, wherein the steps of crushing the flakes or slugs and screening the resulting granules can be repeated one or several times. Preferably, the granulation of the flakes or slugs involves crushing and screening the flakes or slugs, followed by recrushing and rescreening the granules one or two times. The crushing may be carried out by using any conventional crusher, such as a pyramid, roller crusher, or, as typically used for crushing the flakes obtained in roller compaction, an oscillating mill. To crush the slugs, a hammer mill is particularly suitable. For screening, a sieve or screen of a certain mesh size may be used. In the case of the flakes, an oscillating mill fitted with a screen is typically employed for crushing and screening. As mentioned above, many of the commercially available roller compactors contain an integrated granulation system for crushing and screening the flakes and, thus, are most conveniently used for the purpose of the present invention.

Depending on the number of steps of crushing and sieving one or more screens having different mesh sizes, wherein the mesh size decreases form the initial or upper screen to the terminal or lower screen, may be used. A skilled person is readily able to empirically identify appropriate mesh sizes of each of the used screens depending on the particular particle size distribution of the granules required for the intended purpose, such as the preparation of tablets or the use in sachets or capsules. In order to obtain a direct compressible dextrose composition with highly suitable compression properties, the mesh size of the terminal screen, which corresponds to the single screen used in a single-stage granulation process, is 0.1 to 1.8 mm, preferably 0.4 to 1.8 mm. For obtaining granules suited for sachets or capsules filings a terminal screen with a mesh size of 0.4 to 1.8 mm, preferably 0.5 to 1.5 mm, more preferably 0.7 to 1.5 mm, may be used. If the granules are to be compressed into tablets, the terminal screen preferably has a mesh size of 0.1 to 0.8 mm, more preferably 0.2 to 0.7 mm and most preferably 0.4 to 0.6 mm. If more than one screen is used, the initial or first screen preferably has a mesh size of 1.0 to 4.0 mm, preferably 1.0 to 3.0, in particular 2.0 to 3.0 mm.

Preferably, fines that were produced in the compating, crushing and/or screening steps are additionally separated by sieving. The fines may then be recirculated into the roller compactor for re-compression. Typically, particles below 100 $\mu$m are sieved out using sieves of a mesh size of 100 $\mu$m. This is to say, that, if the process includes the step of separating fines, the lower size limit of the granules is determined by the mesh size of the sieve used for sieving out the fines, and the upper size limit of the granule is determined by the mesh size of the above-mentioned terminal sieve.

The direct compressible dextrose composition obtained by the process according to the first aspect of the invention together with one or more active ingredients and, optionally, one ore more additional substances may then be encapsulated to form capsules in a known manner, such as by using a conventional encapsulating machine, or filled into sachets. In particular, the direct compressible dextrose composition of the invention may be formed into tablets by direct compression using a tablet press as further explained below.

In a second aspect, the present invention concerns a direct compression process for preparing a pharmaceutical formulation in tablet form. This process comprises the steps of (a) providing a direct compressible dextrose composition by carrying out the process set forth in claims 1 to 3 and as discussed above, (b) blending the direct compressible dextrose composition with one or more active ingredients to obtain a blend, and (c) compressing the blend into a tablet form.

For compressing the blend into a tablet form any known tablet presses may be used, such as single-station presses, multi-station rotary presses or eccentric presses. Preferably, a rotary tablet press is used, which continuously rotates as part of a die table from the filling position to a compaction position. The particles are compacted between an upper punch and a lower punch to an ejection position, at which the resulting tablet is pushed from the die cavity by the lower punch.

The term "active ingredient", as used herein, refers to any chemical compound or composition having a pharmaceutical effect, in particular a therapeutic or prophylactic effect, and, thus, may encompass, e.g. analgesics, decongestants, expectorants, antihistamines, antitussives, antacids, gastric protectants, diuretics, bronchial dialators, sleep-aids, vitamins, laxatives, antibiotics, antispasmolytics, and the like.

Optionally, the direct compressible dextrose composition is additionally blended with one or more excipients well-known in the art, such as diluents (also called bulking agents or fillers) to provide a quantity of material which can be formed accurately into a compressed tablet, binders to further increase the cohesive properties of the direct compressible dextrose composition, lubricants to further prevent sticking the compressed tablet to the die and punches of the tablet press, disintegrants to facilitate the break-up or disintegration of the compressed tablet after administration, and other compounds, such as preservatives, flavours, antioxidants, surfactants, emulsifiers, and coloring agents. Examples of diluents include lactose, mannitol, sorbitol, microcrystalline cellulose, ethyl cellulose, xylitol, starch, starch derivatives, fructose, calcium carbonate, calcium phosphate, sodium calcium phosphate, and sucrose. Typical binders include hydroxy-propylcellulose, methylcellulose, starch, polyvinylpyrrolidone, hydroxypropylmethylcellulose, gelatin, glycerol, and mixtures thereof. Suitable disintegrants include modified or unmodified starches, clays, crosslinked polyvinylpyrrolidone, modified or unmodified celluloses, gums or alginates. Examples for the lubricants include magnesium stearate and other related stearates, stearic acid, sodium stearyl fumarate, hydrogenated castor or vegetable oil, talc, and polyethylene glycol.

The active ingredient(s) and the excipient(s) may constitute up to 80% by weight of the blend, preferably up to 50% of the blend. The active ingredient may constitute 1 to 80% by weight, preferably 1 to 50% by weight, and the one or more excipients may constitute 0 to 79% by weight, preferably 0 to 49% by weight of the blend. The appropriate effective amount of the particular active ingredient(s) depends on the desired therapeutic response upon administration and can be readily determined by a person skilled in the art considering the bioavailability of the active ingredient, the dosage scheme, the age, sex and weight of the patient, and other factors, as known in the art. The excipient(s) are usually neither required to be present in such high amounts of up to 79% by weight nor 49% by weight due to the excellent properties of the blend, such as compressibility, flowability and friability, imparted by the used direct compressible dextrose composition of the invention. Thus, the amount of the one or more excipient is preferably 40%, 30%, 20%, more preferably 10% or less and most preferably 0 to 5% by weight of the blend.

After having compressed the blend into the form of a tablet, the obtained tablet may be provided with a coat, in particular, if the active ingredient has an objectionable taste. Techniques for tablet coating, such as sugar coating and film coating, are well-known in the art.

The term "tablet form", as used herein, includes any tablet, in particular tablets in any form, shape and of any physical, chemical or sensory property, and tablets for any route of administration, indication and application. The tablets produced according to the invention may be, for example, tablets to be sucked, chewed, dissolved or swallowed. Preferably, the tablet is a chewable tablet. A chewable tablet according to the present invention is a soft tablet where chewing helps to break the tablet particles and release the active ingredient in the mouth before swallowing. A chewable tablet dosage form can be a soft pill, tablet, gum and more recently "chewy squares". The tablet hardness and friability are highly important properties of a chewable tablet comprising active ingredient(s) and having desirable chewability properties. The direct compressible dextrose is particularly suited to meet these needs.

In a third aspect, the present invention relates to a direct compression process for preparing a food formulation in tablet form. The process comprises the steps of (a) providing a direct compressible dextrose composition by carrying out the process as discussed above, (b) blending the direct compressible dextrose composition with one or more food ingredients to obtain a blend, and (c) compressing the blend into a tablet form.

The term "food ingredient", as used herein, refers to any chemical compound or composition which is intended for use in food for humans or animals. Preferably, the food ingredient is for human consumption and approved by the Food and Drug Administration (FDA). The food ingredients for use herein comprise, for example, food supplements, confectioner's sugar, sorbitol, mannitol, starch, starch derivatives, hydrogenated starch hydrolysate, isomalt, isomaltulose, polydextrose or other known sweet materials, and other substances, such as flavours, colorants, preservatives, antioxidants, emulsifiers, and the like.

Preferably, the blend comprises at least 20% by weight of the direct compressible dextrose composition, in particular at least 50%, 60%, 70%, 80% or 90% by weight. The one or more food ingredient may be present in the blend in a total amount of 1 to 80% by weight, in particular 1 to 50% by weight. Preferably, the lower limit of the total amount of the one or more food ingredient is 5%, 10%, 20% or 30% by weight of the blend. The upper limit is preferably 10%, 20%, 30% or 40% by weight of the blend. The amount of the above-mentioned other substances falling within the definition of a food ingredient in the sense of the present invention, is less than 10%, preferably less than 5%, more preferably less than 2% by weight, based on the weight of the blend.

In a fourth aspect of the present invention, there is provided a process for preparing a pharmaceutical formulation in tablet form by dry granulation followed by compression. This process comprises the steps of (a) providing a direct compressible dextrose composition by carrying out the process as discussed above, wherein the starting dextrose composition comprises at least 50% by weight dextrose, 1 to 50% by weight of an active ingredient and 0 to 49% by weight of one or more excipients, and (b) compressing the direct compressible dextrose composition into a tablet form.

The active ingredient(s) and the excipient(s) comprised within the starting dextrose composition are as defined herein in connection with the process of the second aspect of the invention. The appropriate effective amount for a particular active ingredient depends on several factors, as described hereinbefore, and can be readily determined by a person skilled in the art. Preferably, the amount of the one or more excipient is 40% or less, more preferably 30% or less, yet more preferably 20% or less, even more preferably 10% or less and most preferably 0 to 5% by weight of the total weight of the starting dextrose composition.

According to a variation of the process of the fourth aspect of the invention, the direct compressible dextrose composition may be blended with one or more excipients prior to tabletting. Specific examples of optionally used excipients are mentioned herein above. The excipient(s) are preferably added in an amount of less or equal to 40%, 30%, 20%, more preferably less or equal to 10% and most preferably 0 to 5% by weight of the total weight of the blend to be compressed into a tablet form.

The direct compressible dextrose composition obtained in the process of the invention has a favourable dissolution profile and a high flowability, rendering it highly suitable for use in sachets or as capsule fillings. Further, the direct compressible dextrose composition exhibits an increased bulk density and an improved flow and, thus, provides relatively consistent granulation fill volumes for encapsulation. Moreover, the direct compressible dextrose composition has a high compressibility and, thus, is particularly suited for tabletting.

The direct compressible dextrose composition preferably has a volume mean diameter, reference to ISO 9276 part II, of 100 to 800 $\mu$m. For tablet applications, the volume mean diameter is preferably in the range of 150 to 300 $\mu$m, more preferably in the range of 200 to 250 $\mu$m. Such a particle size is suitable to impart the composition with adequate flowability and compressibility. For sachet or capsule applications, the volume mean diameter is larger, e.g. in the range of 300 to 700 $\mu$m. Further, the direct compressible dextrose composition of the invention has preferably a Hausner ratio, an indicator for the flowability which corresponds to the ratio of tapped density to bulk density, of 1.00 to 1.25, typically 1.03 to 1.20, in particular 1.05 to 1.20. The Hausner ratio is determined as described in the example given below.

A direct compressible dextrose composition obtainable by the process of the invention comprising at least 95%, preferably at least 98%, at least 99%, at least 99.5%, or 100% by weight dextrose and being sieved through a sieve with a mesh size of 500 $\mu$m, is characterized by any one of the following parameters: a bulk density of 550 to 750 g/l, preferably 600 to 700 g/l, more preferably 630 to 670 g/l, a tapped density of 700 to 800 g/l, preferably 740 to 780 g/l, a compressibility index of 11 to 17%, preferably 13.0 to 15.5%, and a Hausner ratio of 1.12 to 1.22, preferably 1.14 to 1.20. Further, a tablet prepared from such a direct compressible dextrose composition has a tensile strength at a compression force of 10.0 kN of 0.5 to 2.5 $N/mm^2$, preferably 1.0 to 2.0 $N/mm^2$, a tensile strength at a compression force of 20.0 kN of 1.5 to 4.0 $N/mm^2$, preferably 2.0 to 3.5 $N/mm^2$, a tensile strength at a compression force of 30.0 kN of 2.5 to 5.0 $N/mm^2$, preferably 3.0 to 4.5 $N/mm^2$, and/or a friability of below 1% at compression forces of 20 kN or higher, more preferably a friability of below 1% at compression forces of 10 kN or higher. All these parameters are determined as described in the example as given below.

The direct compressible dextrose composition is particularly suitable to obtain a pharmaceutical formulation.

The pharmaceutical formulation contains the direct compressible dextrose composition of the invention, one or more active ingredients, and optionally one or more excipients. Preferably, the pharmaceutical formulation is in the form of solid dosage forms, such as capsules, tablets, sachets, pills, lozenges, and the like.

Capsules, which contain the granular direct compressible dextrose composition of the invention, include any capsules known in the art, such as gelatine capsules. Methods for encapsulating the direct compressible dextrose composition of the invention are well-known in the art and include, for example, the encapsulation using a Hofliner and Kary or a Bosel GKF encapsulator. The granular direct compressible dextrose composition of the invention may be filled into the body of the capsule dosage form by tamping or dosing and the capsule may be subsequently sealed using a cap.

The tablets are defined as described hereinbefore in connection with the second aspect of the invention. Preferred tablets are chewable tablets. Preferred values for tensile strength and friability are as described above in connection with the fifth aspect of the invention. The hardness of the resulting tablets is strongly influenced by the compression pressure employed. Thus, where it has been desired to produce a softer tablet, the compression pressure is reduced.

Sachets represent a further preferable form of the pharmaceutical formulation, which contains a powder of the direct compressible dextrose composition, the active ingredient(s) and, optionally, the one or more additional ingredients.

The direct compressible dextrose composition is also suitable to produce a food formulation.

Preferably, the food formulation is a chewable food formulation. A chewable food formulation is a formulation where chewing helps to break up the food formulation and to release single constituents contained herein before swallowing. The food formulation preferably present in the form of tablets, lozenges, lollipops and the like. Preferably, the tablet is a confectionary tablet. Further, the tablet is preferably a chewable tablet as defined herein above. Particularly preferred for use within the present invention is a chewable confectionary tablet. An example thereof is a chocolate based tablet.

Finally, the direct compressible dextrose composition obtainable by the process described above is suitable for preparing a pharmaceutical composition or a food formulation, respectively.

The present invention will now be further described by the following figures and examples.

FIGURES

Fig. 1 shows microscopic pictures of pure dextrose before RC (upper picture) and of dextrose after RC (<500 $\mu$m) (lower picture).

Fig. 2 is a diagram showing the compression profile of tablets of pure dextrose before RC (●) and RC dextrose (<500 $\mu$m) (■).

Fig. 3 is a diagram showing the friability as a function of compression force of tablets of pure dextrose before RC (●) and RC dextrose (< 500 $\mu$m) (■). The horizontal solid line denotes the friability limit according to Ph. Eur. V, USP 29/NF 24.

EXAMPLE

In the following example pure dextrose and a blend of dextrose with vitamin C were subjected to roller compaction (RC) followed by granulation, and the resulting RC granules were compared with non roller compacted pure dextrose in terms of particle size distribution, structure, density and flowability. Further, compressed tablets were prepared from these formulations and compared with each other in terms of hardness and friability.

The obtained results show that the roller compaction of both pure dextrose and the blend of dextrose with vitamin C results in granules having an improved flowability and a higher compressibility compared to dextrose before RC. Tablets prepared from these granules showed a significantly increased hardness, and an improved and low friability, and no capping was observed.

1. Experimental procedures

1.1 Materials

a) Raw materials

The following raw materials were used:

1) C*PharmDex 02011 (pharma-grade $\alpha$ dextrose monohydrate, available from Cargill)
2) Fine grade vitamin C (not compressible, available from BASF)

b) Sample dextrose compositions

The following sample dextrose compositions were provided:

1) Pure dextrose (C*PharmDex 02011)
2) 70 wt.% dextrose (C*PharmDex 02011) + 30 wt.% vitamin C

1.2 Preparation of granules and tablets

A roller compactor type WP 120 V Pharma of Alexanderwerk was used for roller compacting the sample dextrose compositions. This roller compactor was fitted with a horizontal feed screw system with vacuum station, vertically positioned rollers, and an integrated, two-stage rotor fine granulator unit.

The powdered sample dextrose compositions were fed to the vertically positioned compacting rolls by the screw feeder and the vacuum of the horizontal feed system to form flakes of a certain size. These flakes were transferred to a two-stage granulating system consisting of an upper and lower sieve, respectively, to form granules. In order to obtain a desired particle size distribution of the obtained granules, the size of the sieves was adapted as described in the following.

An upper sieve having a mesh size of 2.5 mm was used in all experiments and a lower sieve having a mesh size of 1.6 mm and 0.8 mm, respectively, was used to produce granules intended for use in sachets or capsules. To produce granules suited for preparing compressed tablets, a lower sieve having a mesh size of 1.0 mm or 0.8 mm was used. Prior to tabletting these granules, an additional granulation step using a granulator type AR 400 E of Erweka GmbH (Germany) fitted with a sieve of 500 $\mu$m was performed to obtain size-reduced granules which are highly suited for the production of tablets.

After final granulation of the powdered sample dextrose compositions and prior to determining the granule properties, the amount of fines was decreased as much as possible using a sieve of 100 $\mu$m, fitted on a 'Fritsch' Vibratory sieve shaker, for 10 minutes at amplitude 2 mm.

The parameters used for compaction and granulation are shown in Table 1 (Dextr. = dextrose; SF-SP = screw feeder speed; CR-SP = compacting roll speed; HPR = hydraulic pressure; FSIZE = flake size; GR-SP = granulating system speed; MS-US = mesh size of upper sieve; MS-LS = mesh size of lower sieve).

Table 1. Roller compaction and granulation conditions

| | Roller compaction | | | | Granulation | | |
|---|---|---|---|---|---|---|---|
| | SF-SP (rpm) | CR-SP (rpm) | HPR (bar) | FSIZE (mm) | GR-SP (rpm) | MS-US (mm) | MS-LS (mm) |
| Dextr. (for granules) | 50 | 5 | 100 | 2 | 60 | 2.5 | 1.6/0.8* |
| Dextr. (for tablets) | 50 | 5 | 50 | 3 | 60 | 2.5 | 1.0 |
| Dextr. + Vit. C | 35 | 7.5 | 75 | 1 | 60 | 2.5 | 0.8 |
| * granules to be used e.g. in sachets or capsules | | | | | | | |

The obtained granules were then compressed into tablets using a triple punch rotary press type PH 100 - PMA 3 of Korsch GmbH (Germany). The produced tablets were round, flat-faced and had a surface of 1 cm$^2$ and a weight of 350 mg.

### 1.3 Methods for evaluating granule and tablet properties

The granules were characterized by their size distribution, microscopic structure, density and flowability. The following measurement methods were employed.

Size distribution. Size distribution was determined according to the European Pharmacopoeia V Test method 2.9.31 using a laser light particle sizer, type Helos KF - Rodos T4.1, of Sympatec GmbH (Germany). The particle size was analysed by laser light diffraction.

Structure. A Zeiss microscope at magnification 10X was used to take microscopic pictures of pure dextrose before and after roller compaction.

Bulk density. Bulk density was measured according to the European Pharmacopoeia V Test method 2.9.15 - *Apparent Volume.*

Tapped density. Tapped density was determined according to the European Pharmacopoeia V Test method 2.9.15 - *Apparent volume,* using a Stampfvolumeter, model STAV2003, of JEL AG (Germany).

Flowability. Flowability was measured according to the European Pharmacopoeia V Test method 2.9.36 Powder flow - *Compressibility index or Hausner ratio.*

The tablets were characterized by their hardness and friability. For each compression force, 20 tablets for hardness and 19 tablets for friability were analyzed and mean values were calculated. The following measuring methods were employed.

Hardness. Hardness, i.e. the diametral crushing strength, was determined using a conventional pharmaceutical hardness tester (hardness tester model TBH - WS 50, available from Erweka GmbH (Germany)). In order to compare values across different size tablets, the breaking strength was normalized for the area of the break. The normalized value, expressed as N/mm$^2$, is herein referred to as tensile strength (Ts) and calculated as follows:

$$Ts = 2H/\pi TD,$$

wherein H is the hardness, T the thickness and D the diameter of the tablet. For each compression force, 20 tablets were analyzed on hardness (H), thickness (T) and diameter (D).

Friability. Friability measurements were determined according to the European Pharmacopoeia V Test method 2.9.7 *Friability of uncoated tablets.*

### 2. Results

### 2.1 Granule properties

### a) Particle size distribution

The pure dextrose before RC had a volume mean diameter of 176 $\mu$m. In contrast, the dextrose to be used for the production of tablets, which was sieved through a 500 $\mu$m sieve (RC dextrose (<500$\mu$m)), exhibited an increased volume mean diameter of 227 $\mu$m. The RC dextrose sieved through a 0.8 mm or 1.6 mm sieve as final granulation (RC dextrose (< 1600 $\mu$m) and RC dextrose (< 800 $\mu$m)), intended for use in sachets or capsules, exhibited volume mean diameters of 602 $\mu$m and 402 $\mu$m, respectively.

The addition of 30 wt.% vitamin C to the pure dextrose, followed by RC and sieving using a sieve of 800 $\mu$m (70 wt.% dextrose + 30 wt.% vitamin C (< 800 $\mu$m)) leads to a quite similar particle size distribution compared to RC dextrose (< 800 $\mu$m). A similar result was obtained for the same RC sample dextrose compositions which, however, had been sieved through a sieve of 0.5 mm as a final granulation instead of a 0.8 mm sieve.

### b) Structure

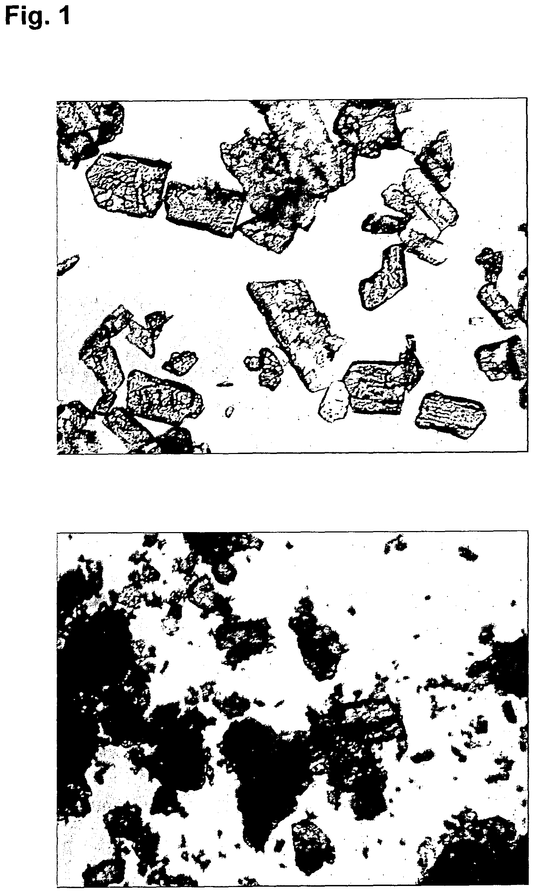

As evident from the microscopic pictures shown in Fig. 1, roller compaction leads to a significantly changed structure of the dextrose before (upper microscopic picture) and after (lower microscopic picture) roller compaction and sieving through a sieve of 500 $\mu$m in terms of surface, volume, shape and size. The substantially altered structure influences both the properties of the granules obtained after RC and the tablets made thereof.

### c) Density and flowability

The bulk density, tapped density, compressibility index and Hausner ratio were determined for RC dextrose, sieved through sieves of 1.6 mm, 0.8 mm and 0.5 mm, and for 30 wt. % vitamin C formulated dextrose sample compositions, sieved through sieves of 0.8 mm and 0.5 mm, respectively. The results are shown in Table 2 below.

Table 2. Density and flowability properties of sample dextrose compositions before and after RC

| | Bulk density (g/l) | Tapped density (g/l) | Compr. Index (%) | Hausner ratio | Scale of Flowability |
|---|---|---|---|---|---|
| PURE DEXTROSE | | | | | |
| After RC (<1.6 mm) | 819 | 853 | 4 | 1.04 | Excellent |
| After RC (<800 μm) | 709 | 804 | 12 | 1.13 | Good |
| After RC (<500 μm) | 650 | 761 | 15 | 1.17 | Good |
| Before RC | 553 | 690 | 20 | 1.25 | Fair |
| FORMULATED DEXTROSE | | | | | |
| After RC of 70 wt.% dextr. + 30 wt.% Vit. C (<800 μm) | 747 | 823 | 9 | 1.10 | Excellent |
| After RC of 70 wt.% dextr. + 30 wt.% Vit. C (<500 μm) | 728 | 823 | 12 | 1.13 | Good |

It was found that the roller compaction of pure dextrose leads to a strong increase of the bulk density and the tapped density. Both the compressibility index and the Hausner ratio are found to be significantly lowered compared to pure dextrose before RC. Low values of compressibility index and Hausner ratio are indicative for a good flowability. In agreement with these findings, the flowability of the RC dextrose was found to be improved compared to the starting dextrose. Further, it was found that the smaller the particle size of the granules, the lower the density and flowability. Similar results were obtained for the dextrose vitamin C formulated granules.

2.2 Tablet properties

a) Hardness

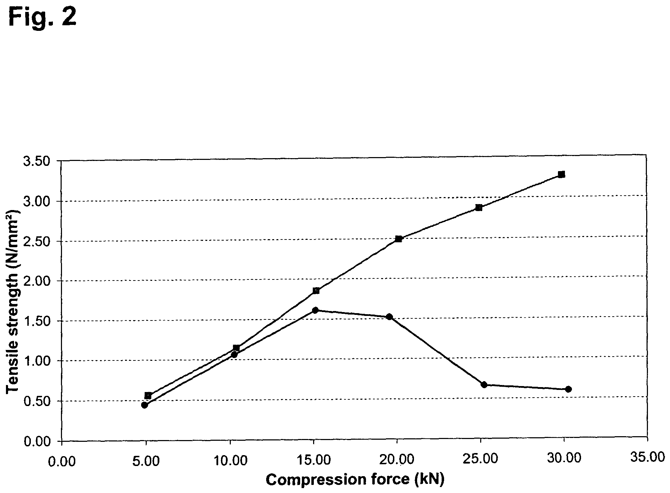

The compression profile, i.e. the tensile strength as a function of compression force, of tablets made of RC dextrose (<500 μm) was found to be much higher than the compression profile of tablets made of non RC dextrose (Fig. 2).
The dextrose tablets, before RC, started to show capping (a tablet defect), which results in a lot of 'incorrect' tablets, at compression forces of 15 kN and higher, and therefore exhibited a decreased tablet hardness. The RC dextrose (<500 μm) did not show this capping and less off-tablets will be produced. The tensile strength of the tablets made of RC dextrose is at least twice that of the tablets made of non RC dextrose. Thus, the RC dextrose powder according to the present invention, is a direct compressible dextrose powder having a higher compressibility than the starting dextrose powder before RC. Similar results were found for tablets made of RC 70 wt.% dextrose + 30 wt.% vitamin C (<500 μm) (results not shown).

b) Friability

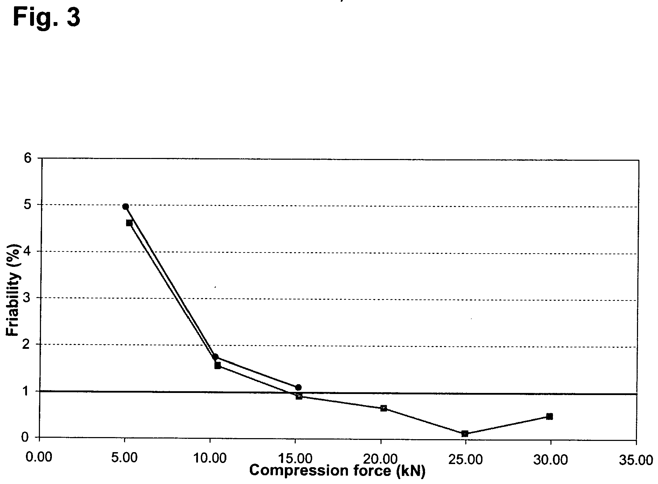

Fig. 3 shows the results of the friability measurements of tablets prepared of non RC dextrose and RC dextrose. The RC dextrose tablets show good friability values at compression forces of around 15 kN or higher. According to the European Pharmacopoeia V the friability of uncoated 350 mg tablets should be below 1%. At compression forces of around 15 kN or higher, the friability of the tablets is below the 1% limit and, thus, complies with the pharmacopoeia. In contrast, the tablets made of non RC dextrose did not go below the 1% limit. In fact, even at compression forces of around 15 kN and higher, no friability values of the tablets could be measured, since the tablets broke up after the friability test. Thus, the friability of tablets produced using the RC dextrose is lower and, thus, more resistant against shipment and abrasion on handling.

**Claims**

1. A dry granulation process for preparing a direct compressible dextrose composition, comprising:

providing a starting dextrose composition in powder form having a moisture content of at least 2%, wherein the starting dextrose composition comprises 50 to 100% by weight α dextrose monohydrate;
compacting the starting dextrose composition by roller compaction using compacting rolls to form flakes or by slugging using a tablet press of single-punch or rotary type to form slugs;
crushing the flakes or the slugs to form granules; and
screening the granules to obtain a direct compressible dextrose composition in the form of granules using a terminal screen with a mesh size of 0.1 to 1.8 mm.

2. The dry granulation process of claim 1, wherein the roll pressure is in the range of 30 to 150 bar.

3. The dry granulation process of claim 1 or 2, wherein the roll gap is in the range of 0.5 to 5 mm.

4. A direct compression process for preparing a food formulation or a pharmaceutical formulation in tablet form, comprising:

   a) providing a direct compressible dextrose composition by carrying out the process of any one of claims 1 to 3;
   b) blending the direct compressible dextrose composition with one or more active ingredients to obtain a pharmaceutical formulation or blending the direct compressible dextrose composition with one or more food ingredients to obtain a food formulation, and
   c) compressing the blend into a tablet form.

5. The process according to claim 4 for preparing a pharmaceutical formulation in tablet form by dry granulation followed by compression, wherein:

   step a) and b) are providing a direct compressible dextrose composition wherein the starting dextrose composition comprises at least 20% by weight dextrose, 1 to 80% by weight of an active ingredient and 0 to 79% by weight of one or more excipients;
   c) compressing the direct compressible dextrose composition into a tablet form.

## Patentansprüche

1. Trockengranulierungsverfahren zum Herstellen einer direktverpressbaren Dextrosezusammensetzung, umfassend:

   Bereitstellen einer Dextrose-Ausgangszusammensetzung in Pulverform mit einem Feuchtigkeitsgehalt von mindestens 2%, wobei die Dextrose-Ausgangszusammensetzung 50 bis 100 Gew.-% eines Dextrosemonohydrats umfasst;
   Kompaktieren der Dextrose-Ausgangszusammensetzung durch Walzenkompaktierung unter Verwendung von Kompaktierwalzen, um Platten zu bilden, oder durch "Slugging" (Tablettenrohlinge) unter Verwendung einer Tablettenpresse vom Schlag- oder Rotationstyp, um Tablettenrohlinge zu bilden;
   Mahlen der Platten oder der Tablettenrohlinge, um Granulatkörner zu bilden; und
   Sieben der Granulatkörner, um eine direktverpressbare Dextrosezusammensetzung in Form von Granulatkörnern unter Verwendung eines terminalen Siebes mit einer Mesh-Größe von 0,1 bis 1,8 mm zu erhalten.

2. Trockengranulierungsverfahren nach Anspruch 1, wobei der Walzendruck im Bereich von 30 bis 150 bar liegt.

3. Trockengranulierungsverfahren nach Anspruch 1 oder 2, wobei der Walzenabstand im Bereich von 0,5 bis 5 mm liegt.

4. Direktverpressverfahren zum Herstellen einer Lebensmittelformulierung oder einer pharmazeutischen Formulierung in Tablettenform, umfassend:

   a) Bereitstellen einer direktverpressbaren Dextrosezusammensetzung durch Ausführen des Verfahrens gemäß einem beliebigen der Ansprüche 1 bis 3;
   b) Mischen der direktverpressbaren Dextrosezusammensetzung mit einem oder mehreren aktiven Bestandteilen, um eine pharmazeutische Formulierung zu erhalten, oder Mischen der direktverpressbaren Dextrosezusammensetzung mit einem oder mehreren Lebensmittelbestandteilen, um eine Lebensmittelformulierung zu erhalten, und
   c) Verpressen der Mischung zu einer Tablettenform.

**5.** Verfahren nach Anspruch 4 zum Herstellen einer pharmazeutischen Formulierung in Tablettenform durch Trockengranulierung, gefolgt von Verpressung, wobei:

Schritt a) und b) eine direktverpressbare Dextrosezusammensetzung bereitstellen, wobei die Dextrose-Ausgangszusammensetzung mindestens 20 Gew.-% Dextrose, 1 bis 80 Gew.-% eines aktiven Bestandteils und 0 bis 79% Gew.-% von einem oder mehreren Hilfsstoffen umfasst;
c) Verpressen der direktverpressbaren Dextrosezusammensetzung zu einer Tablettenform.

**Revendications**

**1.** Procédé de granulation par voie sèche pour préparer une composition de dextrose directement compressible, comprenant :

la fourniture d'une composition de dextrose de départ sous forme de poudre ayant une teneur en humidité d'au moins 2 %, dans laquelle la composition de dextrose de départ comprend 50 à 100 % en poids d'un dextrose monohydraté ;
le compactage de la composition de dextrose de départ par compactage au rouleau à l'aide de cylindres de compactage pour former des flocons ou par briquetage à l'aide d'une presse à comprimés de type mono-poinçon ou rotative pour former des briquettes ;
l'écrasement des flocons ou des briquettes pour former des granules ; et
le tamisage des granules pour obtenir une composition de dextrose directement compressible sous la forme de granules à l'aide d'un tamis terminal ayant une taille de maille de 0,1 à 1,8 mm.

**2.** Procédé de granulation par voie sèche selon la revendication 1, dans lequel la pression du rouleau est dans la plage de 30 à 150 bars.

**3.** Procédé de granulation par voie sèche selon la revendication 1 ou 2, dans lequel l'écartement entre les cylindres est dans la plage de 0,5 à 5 mm.

**4.** Procédé de compression directe pour préparer une formulation alimentaire ou une formulation pharmaceutique sous forme de comprimé, comprenant :

a) la fourniture d'une composition de dextrose directement compressible en mettant en oeuvre le procédé selon l'une quelconque des revendications 1 à 3 ;
b) le mélange de la composition de dextrose directement compressible avec un ou plusieurs principes actifs pour obtenir une formulation pharmaceutique ou le mélange de la composition de dextrose directement compressible avec un ou plusieurs ingrédients alimentaires pour obtenir une formulation alimentaire, et
c) la compression du mélange en une forme de comprimé.

**5.** Procédé selon la revendication 4 pour la préparation d'une formulation pharmaceutique sous forme de comprimés par granulation par voie sèche suivie d'une compression, dans lequel :

les étapes a) et b) fournissent une composition de dextrose directement compressible dans laquelle la composition de dextrose de départ comprend au moins 20 % en poids de dextrose, 1 à 80 % en poids d'un principe actif et 0 à 79 % en poids d'un ou plusieurs excipients ;
c) la compression de la composition de dextrose directement compressible en une forme de comprimé.

**Fig. 1**

# Fig. 2

# Fig. 3

**EP 2 175 740 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005020284 A1
- US 4072535 A
- US 4327077 A
- US 20020122823 A1
- US 20050202084 A1
- US 3619292 A
- US 6451122 B1